# EUROPEAN PATENT SPECIFICATION

(11) **EP 2 322 612 B1**
(45) Date of publication and mention of the grant of the patent: **03.06.2015**
(21) Application number: 09809882.5
(22) Date of filing: 25.08.2009
(51) Int. Cl.: C12N 15/10, C40B 40/06, C40B 50/06

(54) **METHOD FOR PRODUCTION OF cDNA LIBRARY HAVING REDUCED CONTENT OF cDNA CLONE DERIVED FROM HIGHLY EXPRESSED GENE**
VERFAHREN ZUR HERSTELLUNG EINER cDNA-BIBLIOTHEK MIT REDUZIERTEM cDNA-KLONGEHALT AUS HOCH EXPRIMIERTEM GEN
PROCÉDÉ DE FABRICATION D UNE BIBLIOTHÈQUE D ADNc AYANT UNE TENEUR RÉDUITE EN UN CLONE D ADNc DÉRIVÉ D UN GÈNE HAUTEMENT EXPRIMÉ

(30) Priority: 26.08.2008 JP 2008217245
(43) Date of publication of application: 18.05.2011
(73) Proprietor: Hitachi High-Technologies Corporation, Tokyo 105-8717 (JP); Japan as represented by Director General of National Rehabilitation Center for Persons with Disabilities, Tokorozawa-shi Saitama 359-8555 (JP)
(72) Inventor: OHTOKO, Kuniyo, Hitachinaka-shi, Ibaraki 312-0033 (JP); SUGIYAMA Masahide, Tokyo 105-8717 (JP); KATO Seishi, Tokorozawa-shi, Saitama 359-8555 (JP)
(74) Representative: Strehl Schübel-Hopf & Partner
(86) International application number: PCT/JP2009/064755
(87) International publication number: WO 2010/024231

(56) References cited:
- WO-A1-98/39352
- WO-A1-2004/087916
- JP-A- 2000 037 193
- US-A1- 2002 051 970
- US-A1- 2003 211 483
- TRIPATHI ET AL: "Anti HIV-1 virucidal activity of polyamide nucleic acid-membrane transducing peptide conjugates targeted to primer binding site of HIV-1 genome", 5 May 2007 (2007-05-05), VIROLOGY, ACADEMIC PRESS,ORLANDO, US, PAGE(S) 91 - 103, XP022065041, ISSN: 0042-6822 * page 99, column 2, paragraph 2 - page 100, column 1, paragraph 1 *
- LEE REACHING ET AL: "Polyamide nucleic acid targeted to the primer binding site of the HIV-1 RNA genome blocks in vitro HIV-1 reverse transcription", 20 January 1998 (1998-01-20), BIOCHEMISTRY, AMERICAN CHEMICAL SOCIETY, US, PAGE(S) 900 - 910, XP002242997, ISSN: 0006-2960 * page 901, column 1, last paragraph - page 903, column 2, last paragraph *
- IVANOV SERGEI ET AL: "Formation of stable triplexes between purine RNA and pyrimidine oligodeoxyxylonucleotides.", 15 July 2003 (2003-07-15), NUCLEIC ACIDS RESEARCH, VOL. 31, NR. 14, PAGE(S) 4256-4263, XP002666944, ISSN: 0305-1048 * abstract; page 4256, column 2, last paragraph - page 4257, column 1, paragraph 1 * * page 4257, column 2, paragraph 2 - page 4258, column 1, paragraph 1; figure 1 * * page 4261, column 2, paragraph 2 - page 4263, column 1, paragraph 1 *
- NELSON P S ET AL: "Negative selection: a method for obtaining low-abundance cDNAs using high-density cDNA clone arrays", GENETIC ANALYSIS: BIOMOLECULAR ENGINEERING, ELSEVIER SCIENCE PUBLISHING, US, vol. 15, 1 January 1999 (1999-01-01), pages 209-215, XP002953473, ISSN: 1050-3862, DOI: 10.1016/S1050-3862(99)00006-6
- BERTIOLI D J ET AL: "AN ANALYSIS OF DIFFERENTIAL DISPLAY SHOWS A STRONG BIAS TOWARDS HIGH COPY NUMBER MRNAS", NUCLEIC ACIDS RESEARCH, OXFORD UNIVERSITY PRESS, SURREY, GB, vol. 23, no. 21, 1 January 1995 (1995-01-01), pages 4520-4523, XP001069015, ISSN: 0305-1048
- FATIMA BONALDO DE M ET AL: "NORMALIZATION AND SUBSTRACTION: TWO APPROACHES TO FACILITATE GENE DISCOVERY", GENOME RESEARCH, COLD SPRING HARBOR LABORATORY PRESS, WOODBURY, NY, US, vol. 6, 1 January 1996 (1996-01-01), pages 791-806, XP002948254, ISSN: 1088-9051
- BENDER, M. ET AL.: 'Use of a PNA probe to block DNA-mediated PCR product formation in prokaryotic RT-PCR.' BIOTECHNIQUES vol. 42, no. 5, May 2007, pages 609 - 614, XP008135881
- OLDENBURG, R.P. ET AL.: 'Selective amplification of rare mutations using locked nucleic acid oligonucleotides that competitively inhibit primer binding to wild-type DNA.' J. INVEST. DERMATOL. vol. 128, no. 2, February 2008, pages 398 - 402, XP008135588

## Description

### TECHNICAL FIELD

The present invention relates to a method for constructing a cDNA library having a reduced content of cDNA clones derived from a gene that is expressed at high frequency.

### BACKGROUND ART

The full-length sequences of genomic DNAs of various organisms such as humans, mice, rice, nematodes, and yeast have been almost completely determined by genome projects. Now in the post-genome era, research is conducted to examine the kinetics of gene groups in order to macroscopically understand the role of each gene in biological phenomena. Thus, technology for comprehensive analysis of all genes is required.

It is considered that the cells composing a human body have a genome in which about 22,000 types of gene are encoded and several tens of thousands of types of genes are expressed depending on cell type (see International Human Genome Sequencing Consortium, 2004, Nature 432, 931-945). Comprehensive examination of the expression of such genes becomes increasingly important not only for elucidation of biological phenomena, but also for developing pharmaceutical products based on gene information. In particular, comprehensive and detailed analysis of information concerning the protein primary structures encoded by genes requires that DNAs complementary to mRNAs, that is, cDNAs (complementary DNAs), be obtained.

Genes are roughly classified into 3 classes based on expression levels: a group of highly expressed genes expressed at 12,000 or more copies per cell, a group of moderately expressed genes expressed at about 300 copies per cell, and a group of lowly expressed genes expressed at only about 15 copies per cell (see David J. Bertioli., et al., Nucleic Acids Research, 1995, Vol .23, No. 21, 4520-4523). When a cDNA library is constructed using a mixture of mRNAs with different expression levels as a template, the content of the lowly expressed gene clones contained in the cDNA library is lower than that of the highly expressed gene by three or more orders of magnitude. Accordingly, comprehensive analysis of cDNA clones contained in a cDNA library by analyzing partial nucleotide sequences requires unnecessary procedures such as redundant sequencing of clones derived from the highly expressed genes. Many intracellularly expressed genes are expressed at low levels. Hence, methods for reducing the proportion of clones derived from a highly expressed gene have been developed.

One such method uses self-association of the cDNAs derived from highly expressed genes. First, a double-stranded cDNA is prepared from mRNA and then denatured to single strands. Subsequently, an annealing reaction is performed and then single-stranded cDNAs are caused to adsorb hydroxyapatite. They are then collected from a mixture of double-stranded cDNA obtained by re-association and the cDNAs that have remained as single strands. This procedure is repeated several times, and single-stranded cDNAs are collected and then cloned. Specifically, many single-stranded cDNAs are present in the case of highly expressed genes, so that they highly likely form double stranded cDNAs via annealing reaction. However, the content of single-stranded cDNAs among lowly expressed genes is low and the association frequency is low, and thus these single-stranded cDNAs remain as single strands. This method involves collecting single-stranded cDNAs derived from lowly expressed genes that have remained unassociated (see JP Patent Publication (Kokai) No. 4-108385 A (1992)).

Another method is based on hybridization with mRNA. Specifically, mRNA is transcribed from the cDNA of an existing highly expressed gene, biotinylated, and then caused to hybridize to a subject single-stranded cDNA library. cDNA not binding to the magnetic particles is recovered, and cDNA that has reacted with avidin-immobilized magnetic particles so as to bind to the biotinylated mRNA is excluded. Then, the unbound cDNA is cloned and thus a clone of the lowly expressed gene is obtained (see JP Patent Publication (Kokai) No. 2000-325080 A).

A cDNA library having an increased content of lowly expressed genes can be constructed by these methods. However, these methods require an additional step for removing the cDNA of a highly expressed gene after construction of a cDNA library from mRNA, resulting in an increased number of steps compared with those in a general cDNA library construction method. Also, the cDNA of a lowly expressed gene is nonspecifically lost, causing the problem of a reduced yield thereof.

In contrast, a method is proposed by which synthesis of 1 st strand cDNA of a highly expressed gene is suppressed in a step of synthesizing 1st strand cDNA using mRNA as a template. Specifically, in the step of synthesizing 1 st strand cDNA from mRNA using oligo dT primers and reverse transcriptase, a probe specifically binding to a position near the 3' end of the mRNA of a highly expressed gene is caused to also coexist to stop 1 st strand cDNA synthesis and then a probe-bound portion of the mRNA is degraded by RNaseH treatment (see JP Patent Publication (Kokai) No. 2000-37193 A). However, reverse transcriptase has activity of eliminating a probe that has bound upon transcription. Hence, it is thought that when a probe binding to an arbitrarily site of mRNA is used, 1 st strand cDNA synthesis cannot be inhibited. In the above document, a probe binding to a position near the 3' end of mRNA is used.

The use of any such method requires a step of annealing single-stranded cDNAs to form double-stranded cDNA and then integrating the resultant into an appropriate vector. Loss of the cDNA clone of a lowly expressed gene occurs during this process. Hence, it has been difficult to efficiently obtain full-length cDNA clones of a lowly expressed gene. Therefore, a method for efficiently constructing a cDNA library having a low content of a highly expressed gene with fewer steps has been desired.

### DISCLOSURE OF THE INVENTION

### Object to be Achieved by the Invention

An object is to provide a method for efficiently constructing a cDNA library having a reduced content of cDNA derived from a single or a plurality of highly expressed genes in mRNA derived from cells or the like.

### Means for Achieving the Object

The present inventors have discovered that, when cDNA synthesis is performed using a double-stranded DNA primer as described in International Patent Publication WO2004/087916 (Pamphlet), the content of cDNA clones of a target gene can be lowered by causing a probe (for a highly expressed target gene in a nucleic acid sample) to coexist upon an extension reaction of 1st strand cDNA, so as to inhibit the extension reaction of 1st strand cDNA and to inhibit a ring-closing reaction of the conjugate of an mRNA/cDNA heteroduplex derived from the gene and a double-stranded DNA primer. Thus, the present inventors have completed the present invention. Specifically, the present disclosure provides a step for constructing a cDNA library having a reduced content of cDNA derived from the mRNA of a target gene that is a highly expressed gene.

The present disclosure relates to a method for constructing a cDNA library having a reduced content of cDNA clones derived from a target gene, for example, comprising the steps of:
(i) annealing a double-stranded DNA primer to an RNA mixture containing mRNA having a cap structure at the 5' end and then further annealing at least one probe that binds to mRNA of the target gene so as to inhibit a reaction with reverse transcriptase;
(ii) synthesizing 1 st strand cDNA from the double-stranded DNA primer using reverse transcriptase and thus preparing a conjugate of a mRNA/cDNA heteroduplex and the double-stranded DNA primer; and
(iii) ligating the 3' end to the 5' end of the DNA strand containing the cDNA of the conjugate of the mRNA/cDNA heteroduplex and the double-stranded DNA primer using ligase for circularization.
A highly expressed gene can be used as a target gene and a target gene can be selected based on a gene database.

The mRNA having the cap structure in step (i) is contained in a cell extract, for example. The primer sequence of a double-stranded DNA primer to be used herein contains a sequence complementary to the poly (A) sequence of mRNA having a cap structure, for example. Also, as a ligase, T4RNA ligase can be used, for example.

The above method may further comprise, between step (ii) and step (iii), step (ii') of cleaving the conjugate of the mRNA/cDNA heteroduplex and the double-stranded DNA primer with a restriction enzyme, so as to generate a 5' protruding end or blunt end of the double-stranded DNA primer.

Also, the above method may further comprise, following step (iii), step (iv) of substituting the RNA strand of the conjugate of the mRNA/cDNA heteroduplex and the double-stranded DNA primer with a DNA strand.

Also, in the above method, the double-stranded DNA primer may contain a replication origin, or a replication origin and a cDNA expression promoter. The probe that binds to the mRNA of a target gene so as to inhibit a reaction with reverse transcriptase is, for example, an oligonucleotide containing non-natural nucleic acids with a melting temperature (Tm value) higher by 2°C or more than that of a complementary sequence of a partial sequence of the target gene, in which 2 or more consecutive nucleotides from the 5' end side are composed of non-natural nucleic acids. As non-natural nucleic acids, locked nucleic acids, polyamide nucleic acids, or bridged nucleic acids (BNAs) can be used. Probes that bind to the mRNAs of target genes so as to inhibit a reaction with reverse transcriptase may target the mRNAs of different types of target gene.

Furthermore, the present disclosure provides, for example, a probe having a sequence complementary to an mRNA sequence. Specifically, the probe comprises an oligonucleotide that is designed so that the 3' end has a structure modified so that it does not serve as the initiation point for an extension reaction with reverse transcriptase, 2 or more consecutive nucleotides from the 5' end are non-natural nucleic acids, and the Tm value of the nucleotide sequence of the entire probe is higher by 2°C or more than that of a nucleotide sequence composed of only natural nucleic acids,

An example of the probe is used for constructing a cDNA library having a reduced content of cDNA clones derived from a target gene and binds to the mRNA of the target gene so as to inhibit a reaction with reverse transcriptase.

Furthermore, the present disclosure provides, for example, a reagent kit for producing a cDNA library containing the above probe, a double-stranded DNA primer, reverse transcriptase, and T4 RNA ligase.

In addition, in the present invention, the term "double-stranded DNA primer" refers to a primer in which the 3' end of one DNA strand of the double-stranded DNA protrudes and the nucleotide sequence (primer sequence) of the protruding portion has a nucleotide sequence complementary to the template mRNA sequence. This protruding portion hybridizes to template mRNA and functions as a primer when 1 st strand cDNA is synthesized with reverse transcriptase. For cDNA synthesis, a double-stranded DNA primer wherein the sequence of such a protruding portion comprises 30-70 oligo dTs is used.

### Effects of the Invention

According to the present invention, a cDNA library having a reduced content of cDNA derived from the mRNA of a target gene can be provided using fewer steps than conventional methods. When a highly expressed gene is used as a target gene, the content of a lowly expressed gene becomes relatively high among the prepared cDNA clones. Thus, the complete gene information of lowly expressed genes can be efficiently obtained. Moreover, the present invention enables the cloning of the cDNA of a gene that is expressed in trace amounts and thus is cloned with difficulty by a conventional method. These genes that are expressed at low expression levels can be used as target genes for gene diagnosis for diseases or development of pharmaceutical products.

### BRIEF DESCRIPTION OF THE DRAWINGS

Fig. 1 shows basic procedures of the method for cDNA synthesis using a method for inhibiting the extension of 1st strand cDNA according to the present invention.
Fig. 2 shows the contents of genes existing in cDNA libraries constructed from RNA samples used in the examples.
Fig. 3 shows the sequences of probes used for examination.
Fig. 4 is a table showing the contents of Albumin 1 and Major urinary protein 2 in each library, as determined by hybridization.
Fig. 5 is a table showing the contents of Albumin 1 and Major urinary protein 2 in each library, as determined by sequencing of the 5'-terminal nucleotide sequence.

### BEST MODE OF CARRYING OUT THE INVENTION

This description includes part or all of the contents as disclosed in the description and/or drawings of Japanese Patent Application No. 2008-217245, which is a priority document of the present application.

Regarding the cDNA library having a reduced content of target gene-derived cDNA clones, the cDNA library is constructed by a cDNA synthesis method using a double-stranded DNA primer as described in International Patent Publication WO2004/087916 (Pamphlet) resulting in a reduced content of a specific target gene-derived cDNA. The cDNA synthesis method is a method for synthesizing cDNA having a continuous sequence from a nucleotide adjacent to the cap structure of mRNA, for which total RNA of several micrograms can be used as a starting material. Also, cDNA can be synthesized with fewer steps without using PCR. The cDNA synthesis method is further characterized in that a full-length cDNA having the nucleotide sequence corresponding to the full-length sequence ranging from the 5'-terminal cap structure addition site to the 3' terminal poly (A) tail of a complete mRNA guaranteed to have a continuous sequence from the nucleotide at the transcription initiation point can be synthesized with a high yield of 90% or more.

As mRNA samples, total RNA isolated from eukaryotic cells can be used. Also, mRNA having a polyA structure, which has been-purified from the total RNA using an oligo dT-bound carrier may be used. Samples containing these mRNAs desirably merely undergo degradation. Specifically, such RNA sample desirably contains complete mRNA having a cap structure at the 5' end and polyA at the 3' end. The term "RNA mixture containing mRNA having a cap structure" in the present invention may refer to an RNA mixture substantially comprising only mRNA having a cap structure and the RNA mixture may contain mRNA and the like lacking a cap structure in addition to mRNA having a cap structure, for example.

Any gene can be selected as a target gene for reduction of the content of cDNA clones thereof in a cDNA library. In view of construction of cDNA libraries containing many lowly expressed genes, which has been performed with difficulty using conventional methods, highly expressed genes are preferred. Here, the term "highly expressed gene" refers to a gene the mRNA of which is contained at a high level in a mRNA sample because of its high expression level. The term "highly expressed gene" also refers to a gene that exists at about 12,000 copies or more per cell, for example. A gene that exists at about 300 copies or more per cell is also included herein. Many highly expressed genes are known as namely housekeeping genes or householding genes. Housekeeping genes are constantly expressed in all cells and are mainly composed of genes encoding proteins required for the survival of cells (e.g., structural proteins, enzymes of energy metabolic systems, and genes involved in mechanisms for protein synthesis, DNA replication, cell division, and the like).

Specifically, genes of such as glyceraldehyde-3-phosphate dehydrogenase (GAPDH), β-actin, a ribosomal protein, histone, phospholipase, a transferrin receptor, HPRT1, and RNA synthetase are known. These housekeeping genes can be selected as target genes. When a specific cell is used, the number or the type of a highly expressed gene differs depending on cells since a specific gene is specifically expressed at a high level in the relevant cell. Examples of such a gene that is specifically expressed at a high level in such a specific cell include albumin in hepatic cells and genes of major urinary proteins. These highly expressed genes can be selected with the use of public databases of existing genes. For example, through the use of the Bodymap (http://bodymap.ims,u-tokyo.ac.jp) database for genes expressed in each human or mouse tissue, information of highly expressed genes can be obtained for every tissue. Also, if no information exists in the existing databases, a gene expression level in an mRNA sample can be found by a method for obtaining the information of gene expression, such as an EST analysis method, a SAGE method, and a DNA array method. Hence, the gene expression level in a mRNA sample used herein can be found. A list is prepared by numbering genes based on their expression levels and then highly expressed gene candidates to be eliminated are selected. A plurality (e.g., 2 or more types) of target genes are applied in most cases. For example, based on information based on the above database, 2 to 10, 2 to 50, 2 to 100, 2 to several hundred, 2 to 1000, 2 to several thousand, or an even higher number of target genes are selected, in descending order of expression levels.

A cDNA library having a reduced content of a highly expressed gene can be produced by the method of the present invention. Specifically, a cDNA library having an improved content of a gene, number of copies of which is about 12,000 per cell or less, or is preferably about 300 per cell or less, can be produced.

A probe that binds to the mRNA of a target gene has a property of blocking the extension of 1 st strand cDNA to be synthesized by reverse transcriptase in the 5' end direction on the mRNA strand.

The probe has the following characteristics.

### (1) Basic structure

The basic structure of the probe is an oligonucleotide having a sequence complementary to a partial sequence of the mRNA sequence of a target gene, wherein the 3' end is modified so that it does not serve as an initiation point for an extension reaction with reverse transcriptase. For example, the probe has a structure of dideoxyribonucleic acid (ddNTP) lacking a hydroxyl group or has a structure in which a hydroxyl group of a nucleotide at the 3' end is bound to a compound such as biotin.

### (2) Binding position

The nucleotide sequence of mRNA of a target gene to which a probe complementarily binds is designed based on nucleotide sequence information according to actual data or public database of subject gene sequences. At this time, a binding site for a probe is preferably located in a common region among variants since it is known that a plurality of transcript variants are generated from a single gene locus due to differences in promoter, splicing, poly (A) addition site, and the like. (3) Nucleotide length of nucleotide sequence of complementary strand

The nucleotide length of a nucleotide sequence of a complementary strand is not particularly limited, as long as it is sufficient for stable binding to the mRNA of a target gene with a length of 20 to 50 nucleotides, preferably of 20 to 40 nucleotides, and further preferably of 20 to 35 nucleotides.

### (4) Introduction of non-natural nucleic acid

The term "non-natural nucleic acid" refers to a nucleic acid having a structure lacking natural acids, but having nucleotides with structures that are absent in the nature. Introduction of one or a plurality of nucleotides that are absent in natural nucleic acids into a probe refers to introduction of a non-natural nucleic acid(s). The probe is an oligonucleotide containing non-natural nucleic acids.

When non-natural nucleic acids are introduced into a probe, Tm value for the probe is preferably determined to be higher by 2°C or more than the Tm value for a nucleotide sequence that is complementary to a partial sequence of the target sequence and is composed of natural nucleic acids. Also, even higher Tm value is more preferable. Also, Tm value for a probe must be higher than the temperature at which 1 st strand cDNA is synthesized in step (ii). The Tm value for a nucleotide sequence composed of natural nucleic acids can be calculated by a known method. The probe is designed in such a manner so that after the probe has once annealed to the mRNA of a target gene, the probe is not easily separated, resulting in a greater effect of suppressing the extension reaction. Any nucleic acid may be used as a non-natural nucleic acid, as long as it complementarily binds to mRNA. Polyamide nucleic acids or locked nucleic acids, bridged nucleic acids (BNAs), or the like are preferably used, for example. Here, the term "polyamide nucleic acid" refers to a DNA analog containing neutral-amide main-chain bonding (e.g., Nielsen et al., Science 254: 1497, 1991). The term "locked nucleic acid" refers to a bicyclic nucleic acid analog in which an O-methylene (oxy-LNA), an S-methylene (thio-LNA), or an NH₂-methylene component (amino-LNA) binds to a furanose ring at positions 2' and 4'. The term "bridged nucleic acid (BNA)" refers to an artificial synthetic nucleic acid prepared via cross-linking of natural nucleic acid molecules (e.g., JP Patent Publication (Kokai) No. 10-1935098 A (1998)). When a locked nucleic acid is used, the Tm value resulting from the mixing of a natural type and a non-natural type can be predicted by referring to Niels Tolstrup's report (Niels Tolstrup et al., Nucleic Acid Research, 2003, 31, 3758-3762), for example. When a non-natural nucleic acid is introduced, preferably 2 or more nucleotides and further preferably 3 or more nucleotides from the 5' end of the probe are non-natural nucleic acids. Also, non-natural nucleic acids to be introduced are preferably continuous. The upper limit of the number of non natural nucleic acids to be introduced is not limited, but is preferably 10 or less and further preferably 5 or less.

A probe composed of such natural and non-natural nucleic acids can be chemically synthesized by a conventional method. Such probe to be used herein with a general degree of purification can be used.

When a plurality of target genes are present, a plurality of probes are used so that they can correspond to all of these target genes. The total number of probes to be used herein is at least the same as that of target gene types. A plurality of probes differing in positions of partial sequences may be used for one target gene.

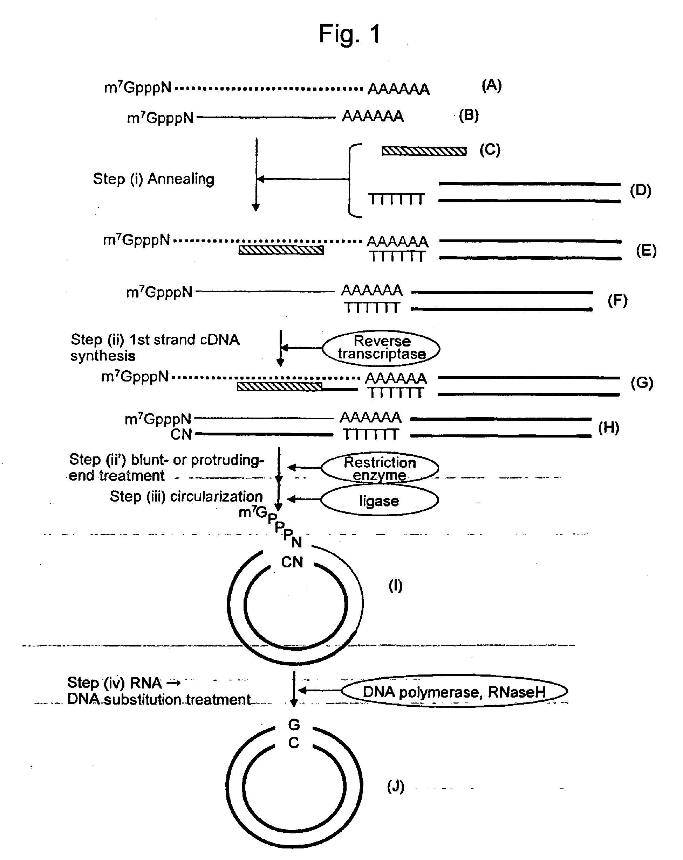

Steps of the present invention will be explained according to Fig. 1.

In step (i), a probe (C) binding to target gene mRNA (A) and a double-stranded DNA primer (D) having oligo dT is annealed to a sample RNA containing the target gene mRNA (A) and non-target gene mRNA (B). Therefore, a target gene mRNA (E) to which the probe (C) and the double-stranded DNA primer (D) are bound is generated, and a non-target gene mRNA (F) to which the double-stranded DNA primer (D) is bound is generated. cDNA can be synthesized with even less than 1 µg of such a sample RNA, but preferably 1 µg or more of a sample RNA is used. A single or 2 or more types of target gene can be used. Hence, a required number of types of the probe (C) having the above characteristics is prepared in accordance with the number of types of target genes. The amount of the probe (C) to be used herein is preferably 50-100 times (in terms of molar ratio) the amount of mRNA (A) or (B), but is not particularly limited thereto. Also, the number of consecutive dTs composing the sequence of the above double-stranded DNA primer (D) preferably ranges from 30 to 70. The double-stranded DNA primer (D) may be such primer containing a replication origin, or a replication origin and a cDNA expression promoter, but the examples thereof are not particularly limited thereto. A restriction enzyme site may be introduced in advance into the double-stranded DNA primer in accordance with the intended use of the cDNA. Annealing temperature may be determined based on the Tm value of the probe (C), so that it is lower than the Tm value of the probe (C).

Probe design and annealing conditions can be adequately determined depending on the types of target gene.

In step (ii), reverse transcriptase is caused to act on the target gene mRNA (E) to which the probe (C) and the double-stranded DNA primer (D) are bound, and the non-target gene mRNA (F) to which the double-stranded DNA primer (D) is bound, so that 1 st strand cDNA complementary to the mRNA in the direction from the 3' end to the 5' end is synthesized. Preferable reverse transcriptase lacks endogenous RNaseH activity. In the case of the target gene mRNA (E) to which the probe (C) and the double-stranded DNA primer (D) are bound, 1 st strand cDNA extension stops before the probe, resulting in an incomplete mRNA/DNA heteroduplex (G) that does not reach the 5' end of the mRNA. Meanwhile, in the case of the non-target gene mRNA (F) to which the double-stranded DNA primer (D) is bound, a mRNA/DNA heteroduplex (H) is formed in which a complementary strand DNA is completely synthesized so as to reach the 5' end. Therefore, 1st strand cDNA is synthesized from only the non-target gene mRNA. In step (ii), a mRNA/cDNA heteroduplex-to-double-stranded DNA primer conjugate is formed. In the conjugate, one end of a cDNA strand of the mRNA/cDNA heteroduplex and one end of a single strand of the double-stranded DNA primer are linked. Also, the cDNA of the mRNA/cDNA heteroduplex generated in step (ii) is complementary to mRNA having a cap structure and is a cap-consecutive cDNA containing dC or 5'-dC(dA)n-3' added to the 3' end or a mRNA-derived cap non-consecutive cDNA having no cap structure.

In step (ii'), other end of the double-stranded DNA primer ligated to the mRNA/DNA heteroduplex is blunt-ended or is preferably treated to have the 5' protruding end through cleavage with a restriction enzyme. In this case, it is required to cause the terminal region of a double-stranded DNA primer to have a restriction enzyme site in advance. In addition, this step can be omitted. Implementation of this step can lead to a significant decrease in the background composed only of a vector contained in a cDNA library.

In step (iii), the mRNA/DNA heteroduplex-to-double-stranded DNA primer conjugate wherein other end of the double-stranded DNA primer has been blunt-ended or treated to have a 5' protruding end is circularized with ligase. As ligase, various types of DNA ligase or RNA ligase can be used and T4RNA ligase is preferably used. A complete non-target gene mRNA/DNA heteroduplex (H), in which the 1 st strand cDNA extends to the 5' end of mRNA, is circularized with ligase. However, an incomplete target gene mRNA/DNA heteroduplex (G), in which 1st strand cDNA extension is not completed, is not circularized.

In step (iv), in the thus circularized non-target gene mRNA/DNA heteroduplex (H), the mRNA strand is degraded with RNaseH and the RNA strand is further substituted with a DNA strand with DNA polymerase and DNA ligase. Accordingly, non-target gene cDNA (J) is synthesized.

If the double-stranded DNA primer (D) contains a replication origin and a drug resistance gene, step (v) is carried out so that transformation into *Escherichia coli* or the like is performed and then the RNA strand may be substituted with a DNA strand in an *in vivo* reaction.

The thus obtained non-target gene double-stranded cDNA may be cloned into a vector. For example, after cleavage at a restriction enzyme site provided in a double-stranded DNA portion, the resultant may be inserted into a plasmid vector, a phage vector, or the like. At this time, through the use of a vector primer as a double-stranded DNA primer,-a step of insertion into another vector can be omitted. A vector primer can be prepared by restrictively cleaving a circular vector DNA at an appropriate cloning site and then ligating a primer sequence complementary to a partial sequence of mRNA to the 3' end, so as to form a 3' protruding end.

A cDNA library obtained by the above method for constructing a cDNA library has a reduced content of target gene-derived cDNA clones. The content of individual target gene-derived cDNA clones accounts for 2% or less and preferably 1 % or less of the total number of cDNA clones. Also, the percentage by which the number of target gene-derived clones is reduced when a probe has been added to the target gene is 60% or more, preferably 70% or more, and further preferably 80% or more. When a target gene is a highly expressed gene, the above cDNA library mainly contains lowly expressed gene-derived cDNAs. In addition, the cDNA library includes clones containing cap-consecutive cDNA with an extremely high probability of 60% or more, preferably 75% or more, further preferably 90% or more, and most preferably 95% or more.

A kit for constructing a cDNA library comprises the probe to be annealed to a target gene and reagents for synthesis of other cDNAs. Examples of reagents for cDNA synthesis include a double-stranded DNA primer, reverse transcriptase, and T4 RNA ligase.

### Examples

Next, the invention will be described more specifically by referring to examples. The present invention is not limited to the examples. In addition, basic procedures concerning DNA recombination and enzyme reactions were carried out according to the literature (Sambrook and Maniatis, in Molecular Cloning - A Laboratory Manual, Cold spring Harbor Laboratory Press, New York, 1989). Restriction enzymes and various modification enzymes used herein were those produced by Takara Shuzo Co., Ltd., unless otherwise specified. Buffer compositions and reaction conditions for each enzyme reaction were employed according to relevant instructions.

### [Example 1]

### Suppression of cDNA extension reaction via probe binding

### (1) Determination of target gene

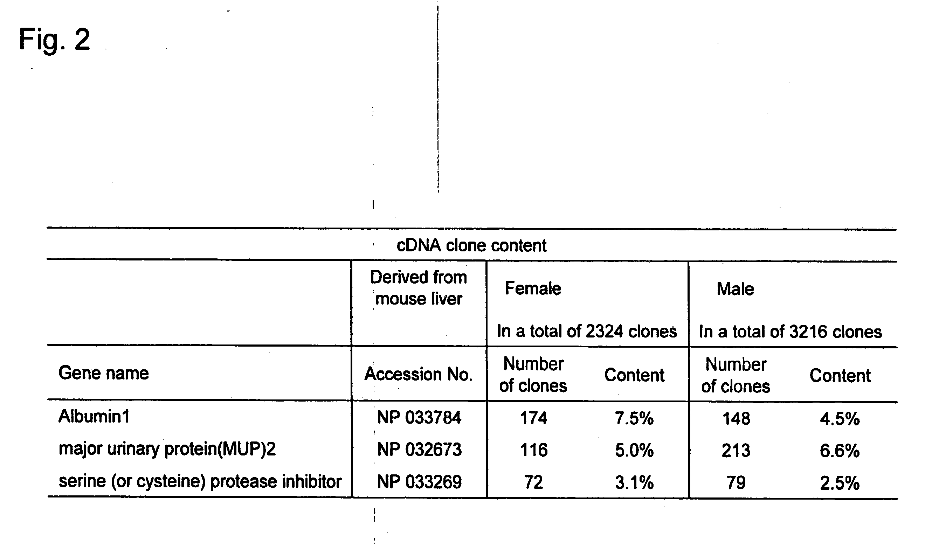

The following experiment was conducted to determine target genes with which experimental effects can be easily confirmed because of their high expression levels. cDNA synthesis was carried out using total RNA (10 µg each) obtained from a male or female mouse liver and 300 ng of pGCAP10 vector primer (International Patent Publication WO2004/087916 (Pamphlet)), so that cDNA clones were obtained. The preparation method was carried out according to description in International Patent Publication WO2004/087916 (Pamphlet). The 5'-end nucleotide sequences of the thus obtained cDNA clones were analyzed. The thus sequenced 3,217 clones derived from a male mouse liver and the thus sequenced 2,325 clones derived from a female mouse liver were subjected to BLAST search using a GenBank nucleic acid database. As a result, a Major Urinary Protein 2 (MUP2) gene was observed to exhibit the highest degree of overlapping (213 clones (6.6%)) among the cDNA clones obtained from a male mouse liver and the Albumin 1 (A1b1) gene was observed to exhibit the highest degree of overlapping (174 clones (7.5%)) among the cDNA clones obtained from a female mouse liver (Fig. 2). Based on these results, the A1b1 gene and the MUP 2 gene were determined as target genes.

### (2) mRNA preparation

T7 RNA polymerase promoter is located upstream of the cDNA cloning site of the pGCAP10 vector and a *Not* I site is located downstream of the same. Hence, the thus obtained cDNA clones of the Alb1 gene and the MUP2 gene were linearized via *Not* I digestion. With the use of the resultants as templates and T7RNA polymerase, mRNA was prepared using an *in vitro* transcription kit (Ambion).

### (3) Probe design

Probes for stopping a reverse transcription reaction, which is cDNA synthesis reaction, were designed.

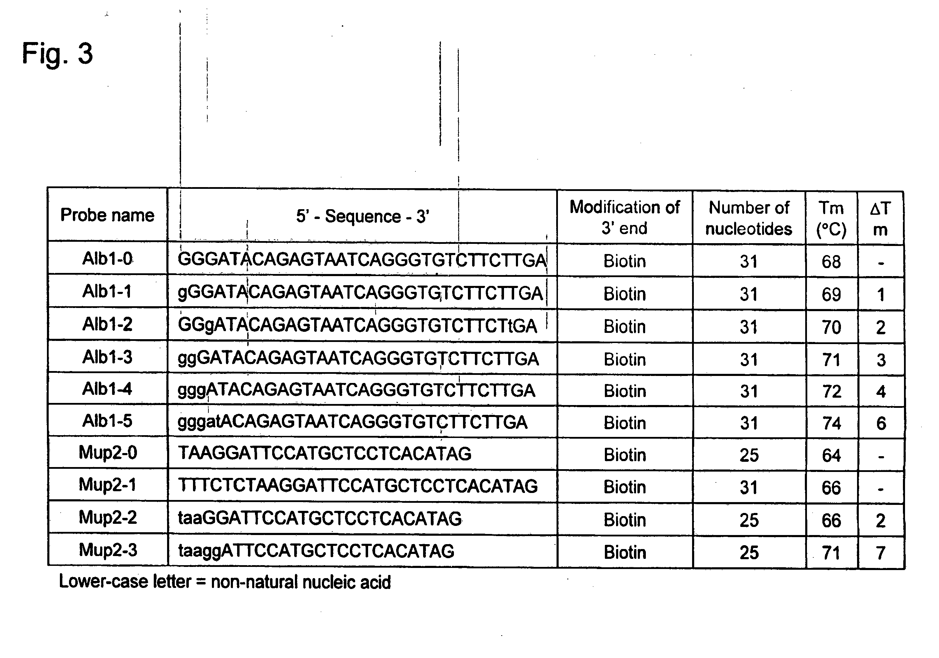

The sequences of translation regions of target gene mRNAs were employed as sequences of the probes. Also, as a non-natural nucleic acid, bridged nucleic acid (BNA) was arranged in the sequence of each probe. There are no examples or detailed information concerning the use of special nucleic acids such as BNA or PNA for stopping reverse transcription or other extension reactions. Examination was carried out by varying the number of BNAs, ranging from 1 to 5. Also, the 3' end was modified with biotin, so as to prevent the probe from functioning as a primer for an extension reaction. The thus designed probe sequences are as shown in Fig. 3. In the sequences in Fig. 3, lower-case letters indicate bridged nucleic acids. The sequences represented by Alb1-0 and Mup-0 are natural nucleic acids. SEQ ID NOS: 1, 2, and 3 in the Sequence Listing represent the sequences of Alb1-0, Mup2-0, and Mup2-1, respectively. Also, we consigned probe synthesis to Gene Design Inc.

### (4) Verification of effect of suppressing reaction

The effect of suppressing the reverse transcription reaction was examined using the probes prepared in (3) above. Probes having "Alb1" in their names were added when Alb1 mRNA was used as a template and probes having "MUP2" in their names were added when MUP2 mRNA was used as a template. cDNA synthesis was carried out for each case. As each reaction solution, a total of 13 µl of a reaction solution was prepared by mixing mRNA (500 ng), poly dT20 primer (100 pmol), dNTP (10 nmol), and a probe (100 pmol) of (3) above. A control reaction solution containing no probe of (3) above was prepared. These reaction solutions -were each maintained at 65°C for 5 minutes and then rapidly cooled on ice for 2 minutes. SuperScript II RNase H-reverse transcriptase (Invitrogen Corporation) (200 U), an attached reaction buffer (4 µl), DTT (100 nmol), and ribonuclease inhibitor (40 U) were added so that the volume of each reaction solution was 20 µl. After 1 hour of reaction at 42°C, 1st strand cDNA was synthesized. The reaction solutions were subjected to phenol extraction and then an mRNA/cDNA heteroduplex was collected by ethanol precipitation. The resultant was dissolved in water (10 µl) containing RNase A to degrade mRNA and then the length of cDNA strand in each reaction solution was confirmed by agarose gel electrophoresis. As a result, compared with the control, no stopping of reverse transcription reaction was observed for the Alb1-1 probe, Alb1-2 probe, or Mup 2-1 probe. However, cDNA synthesis was decreased by about 88% in the case of the A1b1-5 probe, by about 66% in the case of the A1b1-4 probe, by about 25% in the case of the A1b1-3 probe, and by about 50% in the cases of the Mup 2-3 probe and the Mup2-2 probe. Regarding conditions of the arrangement and number of BNAs contained in each probe required for suppression of a reverse transcription reaction, it was suggested by the experimental results that 3 or more consecutive nucleotides are effective, in the case of GC-rich sequences. Also, the effect of the MUP2 probe containing AT-rich BNA is weaker than that of the A1b1 probe. Hence, it was considered that conditions under which similar effects could be obtained with any sequence should be examined

### (5) Optimization of effect of suppressing reaction

Conditions for suppression of cDNA synthesis by Alb1 mRNA were examined using the A1b1-5 probe. Examination was carried out with regard to the amounts of probes to be used, annealing temperature, and annealing time. Reaction was carried out in the above mentioned reaction system by varying only the amount of the probe to be used. Also, regarding annealing temperature, an attached reaction buffer (4 µl), DTT (100 nmol), and ribonuclease inhibitor (40 U) were added so that the volume of the reaction solution was 19 µl, and then the reaction solution was maintained at 42°C or 57°C for 30 minutes to 2 hours. After rapid cooling, SuperScript II RNase H-reverse transcriptase (Invitrogen Corporation) (200 U) was added and then reaction was carried out at 42°C for 1 hour, so that cDNA was synthesized. The detection method was carried out in a manner similar to that in (4) above. As a result, the effect of suppressing reaction by 95% or more was the highest and exhibited under the following conditions: the amount of the probe used was 200 pmol, the annealing temperature was 57°C, and the annealing time was 2 hours. However, the following 3 conditions were determined as optimum conditions under which the effect of suppressing reaction by 90% or more could be obtained in view of the stability of RNA and the amounts of unbound probes, for example: (i) the amount of a probe to be used ranges from 50 to 100 pmol, (ii) the annealing temperature is 57°C, and (iii) the annealing time is 30 minutes.

Next, the effect of suppressing reaction was examined under the above optimum conditions using the Mup2-3 probe or the Mup 2-2 probe with which an effect equivalent to that in the case of the A1b1-5 probe had not been obtained. The effect of suppressing reaction by 90% or more was exerted in the case of the Mup2-3 probe. However, in the case of the Mup2-2 probe, the effect of suppressing reaction by about 50% was exerted, which was similar to that in the previous case. These results suggested that regarding the effect of suppressing reaction under the optimum conditions employed in this experiment, suppression by 90% or more was observed regardless of sequence as long as a probe containing 5 consecutive BNAs had been used. Also, it was suggested that: a probe that is preferably used for the effect of suppressing reaction has a Tm value (Δ Tm) higher by 2°C (or more) than that of a probe prepared using natural nucleic acids and contains 2 or more consecutive non-natural nucleic acids from the 5' end; and the higher the Δ Tm, the higher the effect of suppressing reaction.

### [Example 2]

### Construction of cDNA library having reduced content of highly expressed gene

### (1) 1 st strand cDNA synthesis

cDNA synthesis was carried out from total RNA using the above probes. The probes used herein were Albl-5 and Mup2-3. Total RNA (10 µg each) obtained from the liver of a male mouse, a pGCAP10 vector primer (JP Patent Publication (Kokai) No. 4-108385 A (1992)) (150 ng), dNTP (25 nmol), and 100 pmol probe were mixed, so that a total of 8 µl of each mixture was prepared. The mixed solution was heated at 65°C for 5 minutes and then ice-cooled for 2 minutes. An attached buffer (4 µl) of SuperScript II RNase H-reverse transcriptase (Invitrogen Corporation) was added and then the solution was maintained at 57°C for 30 minutes. Subsequently, the solution was ice-cooled for 2minutes, mixed with DTT (100 nmol), ribonuclease inhibitor (40 U), and SuperScript II RNase H-reverse transcriptase (Invitrogen Corporation) (200 U), so as to adjust the solution to a total volume of 20 µl. The mixed solution was subjected to 1 hour of reaction at 42°C, so that 1st strand cDNA was synthesized. After phenol extraction for the reaction solution, the conjugate of mRNA/cDNA heteroduplex and the vector primer was collected by ethanol precipitation and then dissolved in 80 µl of water.

### (2) Self ligation

10 × H buffer (TAKARA SHUZO Co., Ltd.) and 50 U of restriction enzyme *Eco*R I (TAKARA SHUZO Co., Ltd.) were added to 80 µl of the solution of the conjugate of a mRNA/cDNA heteroduplex and a vector primer to a total volume of 100 µl, followed by 1 hour of reaction at 37°C. The solution was subjected to phenol extraction, the conjugate of the mRNA/cDNA heteroduplex and the vector primer treated with a restriction enzyme was collected by ethanol precipitation, and then the resultant was dissolved in 24 µl of water. The solution was mixed with a reaction solution (50 mM Tris-HC 1 (pH 7.5), 5 mM MgCl₂, 10 mM 2-mercaptoethanol, 0.5 mM ATP, and 2 mM DTT), 120 U of T4 RNA ligase (TAKARA SHUZO Co., Ltd.) was added, followed by 16 hours of reaction at 20°C. The mRNA/cDNA heteroduplex was ligated to *Eco*R I end of the vector primer for circularization (self ligation reaction). The reaction solution was subjected to phenol extraction, a self ligation product was collected by ethanol precipitation, and then the self ligation product was dissolved in 50 µl of water, so that a cDNA vector solution was prepared.

### (3) Transformation of Escherichia coli

The thus prepared cDNA vector solution (1 µl) was mixed with 20 µl of DH10B Electro-cells (Invitrogen Corporation), so that transformation was carried out by an electroporation method. Electroporation was carried out using MicroPulser (Bio-Rad Laboratories Inc.). The thus obtained transformant was suspended in SOC medium, seeded on LB agar medium containing 50 µg/ml ampicillin, and then cultured at 35°C for 16 to 18 hours, so that cDNA clone-transformed *Escherichia coli* was obtained.

### (4) Calculation of the content by hybridization

The following 4 types of library were constructed.
C: Library (C) to which no probe was added.
Alb: Library (Alb) to which Alb1-5 probe was added.
MUP: Library (MUP) to which Mup2-3 probe was added.
W: Library (W) to which Alb1-5 and Mup2-3 probes were added

To find the content of the A1b1 gene (GenBank Accession No. NP_033784) and the content of the MUP 2 gene (GenBank Accession No. NP_032673) contained in the constructed cDNA libraries, about 4000 clones were sampled from each library and then subjected to colony hybridization.

DIG DNA-labeled probe for hybridization was prepared as follows. The Alb1 gene was digested with *Bst* X I and *Not* I restriction enzymes, so that a 1.1-kbp fragment was generated. Also the MUP2 gene was digested with *Avr* II and *Ava* II restriction enzymes, so that a 0.7-kbp fragment was generated. These fragments were subjected to agarose gel electrophoresis, excised, and then increased in amounts by PCR. Subsequently, the DIG DNA-labeled probe was prepared using a DIG High Prime DNA Labeling and Detection Starter Kit I (Roche Applied Science). Next, about 4000 colonies were transferred from each library to a Hybond N + nylon membrane (Amersham). After alkali fixation, hybridization was carried out. Hybridization and detection were carried out according to protocols using DIG High Prime DNA Labeling and Detection Starter Kit I (Roche Applied Science). Hybridization was carried out at 42°C.

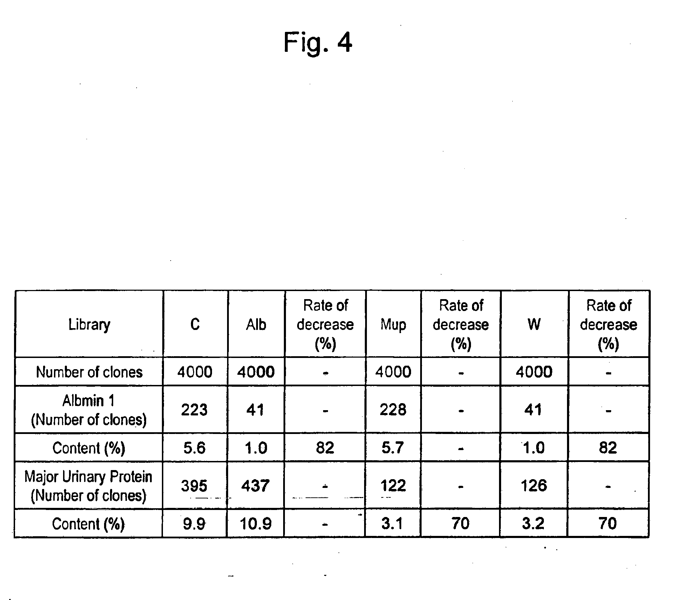

The number and content of clones detected are shown in Fig. 4. The content of A1b1 gene clones decreased from 5.6% to 1.0% in the case of Alb library and also decreased to 1.0% in the case of W library. Therefore, the rate of decrease was 82% in all cases. Also, the content of MUP2 gene clones decreased from 9.9% to 3.1 % in the case of MUP library and decreased to 3.2% in the case of W library. Therefore, the rate of decrease was about 70% in all cases. These results suggest that a decrease by 70% or more can be expected with the addition of the probe. Also, in the case of the W library, the reduction by the same percentage was obtained for each gene even when 2 types of probe had been added.

### (5) Calculation of content via analysis of 5'-end nucleotide sequence

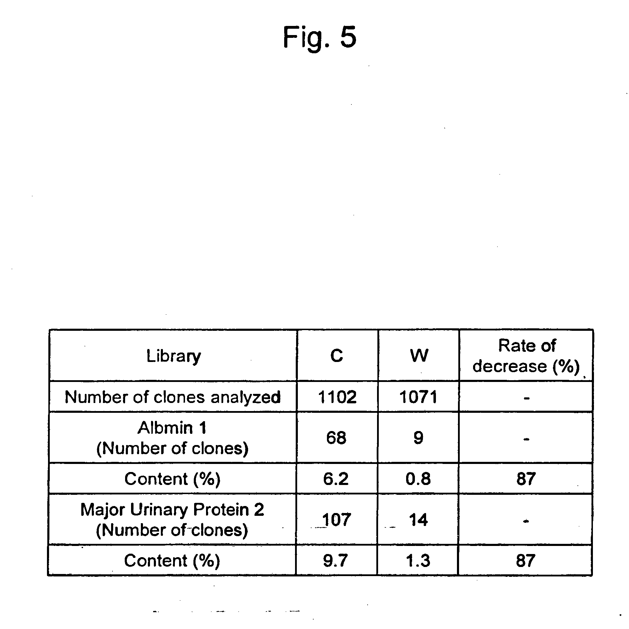

The structural gene region of the nucleotide sequence of the Mup2 gene has 80% or more homology with that of the other Mup genes (Mup1 and Mup3). Therefore, not only the Mup 2 gene, but also the entire Mup group was detected by hybridization. Hence, to find the content more precisely, the nucleotide sequences of about 1000 clones in C and W libraries were analyzed and then numbers of clones, contents, and rates of decreases were found (Fig. 5). As a result, the rate of decrease for Alb1 or for Mup2 was found to be 87%.

As a result of calculation of contents by hybridization and nucleotide sequence analysis above, it was demonstrated that addition of a probe(s) satisfying requirements that should be satisfied by the probes and construction of a cDNA library using a double-stranded DNA primer decrease the content of clones corresponding to the probe(s) by 80% or more and such decreasing effect can be obtained by addition of a plurality of probes.

### Sequence Listing Free Text

SEQ ID NOS: 1-3 Synthesis

## Claims

1. A method for constructing a cDNA library having a reduced content of cDNA clones derived from a target gene, comprising the steps of:
(i) annealing a double-stranded DNA primer to an RNA mixture containing mRNA having a cap structure at the 5' end and then further annealing at least one probe that binds to mRNA of the target gene so as to inhibit the reaction of reverse transcriptase, wherein
the 3' end of one DNA strand of the double-stranded DNA protrudes and the nucleotide sequence of the protruding portion has a nucleotide sequence complementary to the template mRNA sequence, and
the probe that binds to the mRNA of a target gene so as to inhibit a reaction with reverse transcriptase is an oligonucleotide that is designed so that the 3' end has a structure modified so that it does not serve as the initiation point for an extension reaction with reverse transcriptase, 2 or more consecutive nucleotides from the 5' end are non-natural nucleotides, and the Tm value of the nucleotide sequence of the entire probe is higher by 2°C or more than that of a nucleotide sequence composed of only natural nucleotides;
(ii) synthesizing 1st strand cDNA from the double-stranded DNA primer using reverse transcriptase and thus preparing a conjugate of a mRNA/cDNA heteroduplex and the double-stranded DNA primer; and
(iii) ligating the 3' end to the 5' end of the DNA strand containing the cDNA of the conjugate of the mRNA/cDNA heteroduplex and the double-stranded DNA primer using ligase for circularization.

2. The method according to claim 1, wherein the target gene is a highly expressed gene.

3. The method according to claim 1, comprising a step of selecting the target gene based on a gene database.

4. The method according to claim 1, wherein the mRNA having the cap structure is contained in a cell extract.

5. The method according to claim 1, wherein the primer sequence of a double-stranded DNA primer contains a sequence complementary to the poly (A) sequence of mRNA having a cap structure.

6. The method according to claim 1, wherein the ligase is T4RNA ligase.

7. The method according to claim 1, further comprising, between step (ii) and step (iii), step (ii') of cleaving the conjugate of the mRNA/cDNA heteroduplex and the double-stranded DNA primer with a restriction enzyme, so as to generate a 5' protruding end or blunt end of the double-stranded DNA primer.

8. The method according to claim 1, further comprising, following step (iii), step (iv) of replacing the RNA strand of the conjugate of the mRNA/cDNA heteroduplex and the double-stranded DNA primer with a DNA strand.

9. The method according to claim 1, wherein the double-stranded DNA primer contains a replication origin, or a replication origin and a cDNA expression promoter.

10. The method according to claim 1, wherein the oligonucleotide containing non-natural nucleotides is a locked nucleic acid, a polyamide nucleic acid, or a bridged nucleic acid.

11. The method according to claim 1, wherein the probes that bind to the mRNAs of target genes so as to inhibit a reaction with reverse transcriptase target the mRNAs of different types of target gene.

## Patentansprüche

1. Verfahren zum Konstruieren einer cDNA-Bibliothek mit einem verringerten Gehalt an von einem Zielgen abgeleiten cDNA-Klonen, welches die folgenden Stufen umfasst:
(i) das Binden eines doppelsträngigen DNA-Primers an ein RNA-Gemisch, welches mRNA mit einer Deckelungsstruktur am 5'-Ende enthält, und außerdem das Binden mindestens einer Sonde, die an die mRNA des Zielgens bindet, so dass die Reaktion von reverser Transkriptase verhindert wird, wobei
das 3'-Ende eines DNA-Stranges der doppelsträngigen DNA hervorsteht und die Nukleotidsequenz des hervorstehenden Bereiches eine zu der Matrizen-mRNA-Sequenz komplementäre Nukleotidsequenz aufweist, und die Sonde, die an die mRNA des Zielgens bindet, so dass die Reaktion mit reverser Transkriptase inhibiert wird, ein Oligonukleotid ist, das so aufgebaut ist, dass das 3'-Ende eine modifizierte Struktur aufweist, so dass es nicht als Initiationsstelle für eine Extensionsreaktion mit reverser Transkriptase dienen kann,
zwei oder mehr aufeinanderfolgende Nukleotide von dem 5'-Ende nicht-natürliche Nukleotide sind und der Tm-Wert der Nukleotidsequenz der gesamten Sonde um 2°C oder mehr höher ist als derjenige einer Nukleotidsequenz, die nur aus natürlichen Nukleotiden zusammengesetzt ist;
(ii) das Synthetisieren einer cDNA eines ersten Stranges von dem doppelsträngigen DNA-Primer unter Verwendung von reverser Trsanskriptase und somit das Herstellen eines Konjugats aus einem mRNA/cDNA-Heteroduplex und dem doppelsträngigen DNA-Primer; und
(iii) das Ligieren des 3'-Endes an das 5'-Ende des DNA-Stranges, der die cDNA des Konjugats aus dem mRNA/cDNA-Heteroduplex und dem doppelsträngigen DNA-Primer enthält, unter. Verwendung einer Ligase zur Ringbildung.

2. Verfahren nach Anspruch 1, wobei das Zielgen ein hochexprimiertes Gen ist.

3. Verfahren nach Anspruch 1, welches eine Stufe umfasst, in der das Zielgen auf der Grundlage einer Gendatenbank ausgewählt wird.

4. Verfahren nach Anspruch 1, wobei die mRNA mit der Deckelungsstruktur in einem Zellextrakt enthalten ist.

5. Verfahren nach Anspruch 1, wobei die Primer-Sequenz eines doppelsträngigen DNA-Primers eine zu der Poly(A)-Sequenz von mRNA mit einer Deckelungsstruktur komplementäre Sequenz enthält.

6. Verfahren nach Anspruch 1, wobei die Ligase T4-RNA-Ligase ist.

7. Verfahren nach Anspruch 1, das außerdem zwischen der Stufe (ii) und der Stufe (iii) die Stufe (ii') umfasst, in der das Konjugat aus dem mRNA/cDNA-Heteroduplex und dem doppelsträngigen DNA-Primer mit einem Restriktionsenzym gespalten wird, so dass ein 5'-überstehendes Ende oder ein stumpfes Ende des doppelsträngigen DNA-Primers erzeugt wird.

8. Verfahren nach Anspruch 1, das außerdem nach der Stufe (iii) die Stufe (iv) umfasst, in der der RNA-Strang des Konjugats aus dem mRNA/cDNA-Heteroduplex und dem doppelsträngigen DNA-Primer durch einen DNA-Strang ersetzt wird.

9. Verfahren nach Anspruch 1, wobei der doppelsträngige DNA-Primer einen Replikationsursprung oder einen Replikationsursprung und einen cDNA-Expressionspromotor enthält.

10. Verfahren nach Anspruch 1, wobei das Oligonukleotid, das nicht-natürliche Nukleotide enthält, eine gesperrte Nukleinsäure, eine Polyamidnukleinsäure oder eine verbrückte Nukleinsäure ist.

11. Verfahren nach Anspruch 1, wobei die Sonden, die an die mRNAs von Zielgenen binden, um die Reaktion mit reverser Transkriptase zu inhibieren, die mRNAs unterschieldicher Typen des Zielgens als Ziel haben.

## Revendications

1. Procédé de construction d'une bibliothèque d'ADNc ayant une teneur réduite en clones d'ADNc dérivés d'un gène cible, comprenant les étapes qui consistent à :
(i) anneler une amorce d'ADN double brin à un mélange d'ARN contenant un ARNm ayant une structure de coiffe à l'extrémité 5' puis en outre anneler au moins une sonde qui se lie à l'ARNm du gène cible de sorte à inhiber la réaction d'une transcriptase inverse, où
l'extrémité 3' d'un brin d'ADN de l'ADN double brin est saillante et la séquence de nucléotides de la partie saillante possède une séquence de nucléotides complémentaire à la séquence d'ARNm matrice, et
la sonde qui se lie à l'ARNm du gène cible de sorte à inhiber la réaction d'une transcriptase inverse est un oligonucléotide qui est conçu afin que l'extrémité 3' possède une structure modifiée de sorte qu'elle ne serve pas de point d'initiation d'une réaction d'extension avec une transcriptase inverse, que 2 nucléotides consécutifs ou plus de l'extrémité 5' soient des nucléotides non naturels, et que la valeur Tm de la séquence de nucléotides de la sonde entière soit supérieure de 2 °C ou plus à celle d'une séquence de nucléotides composée de nucléotides naturels uniquement ;
(ii) synthétiser un 1^{er} brin d'ADNc à partir de l'amorce d'ADN double brin en utilisant une transcriptase inverse et ainsi préparer un conjugué entre un hétéroduplex d'ARNm/ADNc et l'amorce d'ADN double brin ; et
(iii) ligaturer l'extrémité 3' à l'extrémité 5' du brin d'ADN contenant l'ADNc du conjugué entre l'hétéroduplex d'ARNm/ADNc et l'amorce d'ADN double brin en utilisant une ligase pour la circularisation.

2. Procédé selon la revendication 1, dans lequel le gène cible est un gène hautement exprimé.

3. Procédé selon la revendication 1, comprenant une étape de sélection du gène cible basée sur une base de données de gènes.

4. Procédé selon la revendication 1, dans lequel l'ARNm ayant la structure de coiffe est contenu dans un extrait cellulaire.

5. Procédé selon la revendication 1, dans lequel la séquence d'amorce d'une amorce d'ADN double brin contient une séquence complémentaire à la séquence poly(A) de l'ARNm ayant une structure de coiffe.

6. Procédé selon la revendication 1, dans lequel la ligase est l'ARN ligase de T4.

7. Procédé selon la revendication 1, comprenant en outre, entre l'étape (ii) et l'étape (iii), l'étape (ii') qui consiste à cliver le conjugué entre l'hétéroduplex d'ARNm/ADNc et l'amorce d'ADN double brin avec une enzyme de restriction, de sorte à générer une extrémité saillante 5' ou une extrémité franche de l'amorce d'ADN double brin.

8. Procédé selon la revendication 1, comprenant en outre, après l'étape (iii), l'étape (iv) qui consiste à remplacer le brin d'ARN du conjugué entre l'hétéroduplex d'ARNm/ADNc et l'amorce d'ADN double brin par un brin d'ADN.

9. Procédé selon la revendication 1, dans lequel l'amorce d'ADN double brin contient une origine de réplication, ou une origine de réplication et un promoteur d'expression d'ADNc.

10. Procédé selon la revendication 1, dans lequel l'oligonucléotide contenant des nucléotides non naturels est un acide nucléique bloqué, un acide nucléique polyamide, ou un acide nucléique ponté.

11. Procédé selon la revendication 1, dans lequel les sondes qui se lient aux ARNm de gènes cibles de sorte à inhiber une réaction avec une transcriptase inverse ciblent les ARNm de différents types de gène cible.


### SEQUENCE LISTING

<110> HITACHI HIGH-TECHNOLOGIES CORPORATION; Japan as Represented by Director General of National Rehabilitation Center for Persons with Disabilities
<120> A method for producing cDNA library wherein the content of cDNA clones derived from highly expressed genes have been reduced
<130> EPA-46698
<150> JP 2008-217245
   <151> 2008-08-26
<160> 3
<170> PatentIn version 3.4
<210> 1
   <211> 31
   <212> DNA
   <213> Artificial
<220>
   <223> Synthetic probe
<400> 1
   gggatacaga gtaatcaggg tgtcttcttg a 31
<210> 2
   <211> 25
   <212> DNA
   <213> Artificial
<220>
   <223> Synthetic probe
<400> 2
   taaggattcc atgctcctca catag 25
<210> 3
   <211> 31
   <212> DNA
   <213> Artificial
<220>
   <223> Synthetic probe
<400> 3
   tttctctaag gattccatgc tcctcacata g 31